# EUROPÄISCHE PATENTSCHRIFT

(11) **EP 1 872 735 B1**
(45) Veröffentlichungstag und Bekanntmachung des Hinweises auf die Patenterteilung: **18.05.2016**
(21) Anmeldenummer: 06013010.1
(22) Anmeldetag: 23.06.2006
(51) Int. Cl.: A61B 34/20, A61B 90/00, A61B 17/00

(54) **Verfahren zum automatischen Identifizieren von Instrumenten bei der medizinischen Navigation**
Method for automatic identification of instruments during medical navigation
Procédé d'identification automatique d'instruments lors de navigation médicale

(43) Veröffentlichungstag der Anmeldung: 02.01.2008
(73) Patentinhaber: Brainlab AG, 85622 Feldkirchen (DE)
(72) Erfinder: Goldbach, Günter, 85457 Wörth/Wifling (DE)
(74) Vertreter: Schwabe - Sandmair - Marx

(56) Entgegenhaltungen:
- EP-A- 1 563 799
- WO-A-2005/067794
- WO-A2-01/54558
- US-A1- 2005 215 888

## Beschreibung

Die Erfindung betrifft ein Verfahren zum Identifizieren eines Instruments für die Navigation mit einem medizintechnischen Navigationssystem. Medizintechnische Navigationssysteme sind bekannt und werden eingesetzt, um behandelnden Ärzten bei Operationen eine visuelle Unterstützung zur Verfügung zu stellen. Dabei wird angezeigt, wo sich Instrumente, die durch ein sogenanntes Trackingsystem positionell bestimmt und verfolgt werden, gegenüber bestimmten Patientenkörperteilen befinden. Die Informationen über die Körperteile werden vorab oder intraoperativ durch bildgebende Verfahren ermittelt, und mit Hilfe einer anfänglichen Registrierungsprozedur werden die Raumpositionen des Patienten, seiner Bilddaten und der im Operationssaal verwendeten Instrumente anfänglich zugeordnet, so dass im Folgenden eine Navigation bzw. eine bildunterstützte Chirurgie durchgeführt werden kann. Ein Navigationssystem, das beispielhaft angeführt werden kann, ist in der DE 19 639 615 C2 beschrieben.

Die WO 01/54558 A2 offenbart ein medizintechnisches Navigationssystem mit mindestens einem Instrument, einem Computersystem und einem Sensorsystem. Das Sensorsystem ist dazu geeignet, die Position des mindestens einen Instruments drahtlos zu erfassen und die Positionsinformation an das Computersystem zu übertragen.

Die US 2005/021,5888 A1 beschreibt eine universelle Trackingvorrichtung für ein medizinisches Instrument, wobei unterschiedliche Geometrien, die durch die Trackinganordnungen definiert werden und bestimmten Instrumenten entsprechend zugeordnet sind vom Navigationssystem erkennbar und registrierbar sind.

Die EP 1 563 799 A1 beschreibt ein Verfahren zum Neukalibrieren oder Neureferenzieren eines Körpers oder Instruments mit einem daran befestigten Referenzstern, wobei die Position des Referenzsterns relativ zum Körper oder Instrument verändert wird und die Veränderung der Position des Referenzsterns relativ zum Körper oder Instrument detektiert wird.

Die WO 2005/067794 A1 beschreibt ein Navigationssystem, bei dem Marker im Bereich des sichtbaren Lichts aufgenommen werden und mit einem Referenzbild verglichen werden.

Das grundlegende Konzept solcher Systeme für die bildgeführte Chirurgie, die Markeranordnungen verwenden, beruht darauf, dass Referenzanordnungen an Instrumenten, Patienten oder behandlungsunterstützenden Geräten angeordnet werden, die als starre Körper ausgebildet sind und Koordinatensysteme definieren. Diese starren Marker-Körper werden in ein Referenzsystem übertragen oder mit einem Referenzsystem in Vergleich gestellt, das durch einen Referenzaufbau vorgegeben wird durch eine absolute Positionsdefinition eines Trackingsystems oder das Konzept, welches für Systeme gemäß der oben bezeichneten Druckschrift, aber auch für andere Navigations- bzw. Trackingsysteme gilt (optische Trackingsysteme, magnetische Trackingsysteme, UItraschall-Trackingsysteme etc.) liegt darin, dass im Navigationssystem vordefinierte, starre Markergeometrien hinterlegt werden. Mit anderen Worten sind dem Navigationssystem vorab schon eine Anzahl von unterschiedlichen und charakteristischen Markeranordnungen bekannt, die nur für eine bestimmte Referenzanordnung (Markergruppe) gelten oder schon einem Instrument zugeordnet sind, dessen Geometrie ebenfalls im Navigationssystem hinterlegt ist. Wenn das Navigationssystem mit Hilfe der Daten aus dem Trackingsystem also eine bestimmte und charakteristische Markeranordnung erkennt, wird bei herkömmlichen Systemen diese Markeranordnung mit vorab in einem Datenspeicher hinterlegten Markeranordnungen verglichen und dann der Referenzanordnung oder dem Instrument zugeordnet, welche bzw. welches dieser Markeranordnung entspricht. Dabei ist es von höchster Wichtigkeit, dass die starren Geometrien, die durch die Markeranordnungen definiert werden, eine sehr hohe Fertigungsgenauigkeit haben, damit die Identifizierung einwandfrei erfolgen kann und sechs Freiheitsgrade für die Ortung des Instruments errechnet werden können (dreidimensionale Position + Ausrichtung an den drei Raumachsen).

Es ist die Aufgabe der vorliegenden Erfindung, ein Verfahren zum Identifizieren eines Instruments bereitzustellen, das nicht mehr von vordefinierten, hinterlegten Markergeometrien und den daran geknüpften Anforderungen abhängig ist. Diese Aufgabe wird erfindungsgemäß durch ein Verfahren gemäß dem Anspruch 1 gelöst. Die Unteransprüche definieren bevorzugte Ausführungsformen der Erfindung.

Die vorliegende Erfindung offenbart ein Verfahren zum Identifizieren eines Instruments für die Navigation mit einem medizintechnischen Navigationssystem, wobei das Instrument mit einer Referenzanordnung versehen ist, die mehrere Marker (A, B, C) umfasst, deren Anordnung an der Referenzanordnung einen starren Körper bildet, wobei die Lage der Marker zueinander im Navigationssystem nicht als charakteristische Anordnung für ein bestimmtes Instrument bekannt ist, gekennzeichnet mit den folgenden Schritten:
- mittels eines dem Navigationssystem zugeordneten medizintechnischen, optischen Trackingsystems wird der Abstand der Marker (A, B, C) zueinander gemessen,
- die Markeranordnung mit dem gemessenen Abstand für die Marker wird als eine zuordnungsfähige Markeranordnung identifiziert;
- die Markeranordnung wird dem Instrument zugeordnet und das Instrument wird vom Navigationssystem identifiziert.

Mit derzeit zur Verfügung stehenden und insbesondere mit hochdynamischen Trackingsystemen ist es möglich, die relativen Abstände der erkannten Marker des Instruments in dem Sichtfeld des Trackingsystems zu berechnen, und dies gilt insbesondere auch für bewegte Markergeometrien. Die Systeme und insbesondere hochdynamische Systeme sind schnell genug, um die räumlichen Positionen der Marker mit einer vorgegebenen Genauigkeit zu erfassen, wobei die Erfassung schneller stattfindet als typische Bewegungen des Instruments bei der Verwendung. Die Verwendung eines solchen hochdynamischen Systems ist nützlich, jedoch nicht unbedingt notwendig, um die vorliegende Erfindung zu implementieren.

Wenn die Marker in ihrer Anordnung an der Referenzanordnung einen starren Körper bilden werden sie immer denselben Abstand zueinander haben, auch wenn das Instrument, das Referenzsystem oder das Trackingsystem bewegt werden. Auf dieser Grundlage ist es möglich, die starren Körper dadurch zu identifizieren, dass man eine Bewegung gegenüber dem Trackingsystem oder gegenüber anderen, schon identifizierten Instrumenten herstellt. Die Erfindung nutzt nun den Umstand, dass die Markeranordnung einen starren Körper bildet, zur Identifizierung des Instruments bzw. der Markeranordnung als zuordnungsfähige Markeranordnung. Mit anderen Worten wird nicht mehr ein Vergleich des erfassten starren Körpers mit vordefinierten, bekannten und im Navigationssystem hinterlegten Markergeometrien durchgeführt, sondern die starren Körper, welche die Markergeometrien bilden, werden durch die Messung der relativen Positionen bzw. Abstände der Marker bestimmt bzw. identifiziert. Das erfindungsgemäße Verfahren nimmt also eine Markeranordnung, die in das Sichtfeld des Trackingsystems eingebracht ist, so wie sie ist zur Kenntnis und ordnet sie als zuordnungsfähige und spezielle Markerkonfiguration oder Markergeometrie ein, die dann einem Instrument zugeordnet werden kann. Hieraus ergeben sich vielfältige Vorteile.

Es wird zum Beispiel möglich, eine große Anzahl unterschiedlicher Starrkörper-Markergeometrien zu verwenden, ohne diese vorab in einer Datenbank hinterlegen zu müssen. Weil die Markergeometrien nicht den Vorgaben der bekannten bzw. hinterlegten oder identifizierbaren Markergeometrien im Navigationssystem entsprechen müssen, ist es nach der Realisierung der vorliegenden Erfindung möglich, auch sehr ungenau gefertigte und kostengünstige, mit Markern versehene Instrumente zu verwenden, weil der exakte Abstand zwischen den Markern für die Identifizierung des Instruments nicht länger relevant ist. Die einzige Voraussetzung für die erfindungsgemäße Identifizierung ist nämlich, dass der Markergeometrie-Starrkörper während der Verwendung des Instruments durch den Chirurgen auch ein Starrkörper bleibt.

Die Erfindung kann die Zuverlässigkeit und die Verwendbarkeit von chirurgischen Navigationssystemen bzw. bildgeführten Chirurgiesystemen aufgrund ihrer geringeren Anforderungen an die Fertigungsgenauigkeit bzw. an die bleibende Form des Instruments stark verbessern bzw. zeitlich ausdehnen. Ein weiterer Vorteil der nicht mehr vorhandenen Anforderungen an die Fertigungstoleranzen liegt darin, dass relativ billig herstellbare Einweg-Instrumente verwendet werden können, die auch für den Einsatz bei sehr kritischen Anwendungen, wie zum Beispiel bei Kreuzfeld-Jakob-Krankheiten verwendbar sind. Gleiches gilt für Anwendungen, die mit geringem Budget zu verwirklichen sind, wo eine Sterilisierung der Instrumente nicht erschwinglich ist.

Bei einer Ausführungsform des erfindungsgemäßen Verfahrens wird das Instrument bei der Messung des Abstandes der Marker zueinander bewegt, um die Markeranordnung zu identifizieren. Auch das Trackingsystem kann bewegt werden, wichtig ist eine Relativbewegung zwischen Markeranordnung und Trackingsystems.

Erfindungsgemäß wird das Positionsverhältnis des funktionalen Abschnitts des Instruments, insbesondere der Spitze des Instruments, zu der Markeranordnung durch eine Bewegung des Instruments bei feststehendem funktionalen Abschnitt, insbesondere bei feststehender Spitze durch das Tracking der Marker bestimmt. Das Positionsverhältnis kann hierbei als räumliche Relation von starrer Markeranordnung und funktionellem Abschnitt (Spitze) definiert werden. Die Abstandsmessung kann dabei gleichzeitig mit der Bestimmung des Positionsverhältnisses oder nach dieser Bestimmung erfolgen. Weil die Marker bei feststehender Spitze bzw. bei feststehendem funktionalen Abschnitt des Instruments eine Kreis- oder Kugelbewegung um die Spitze machen werden, kann das Zentrum dieser Kreis- oder Kugelbewegung ermittelt werden, weil auch die Bahnen der Marker über das Trackingsystem ermittelt werden. Deshalb ist es nicht notwendig, dass der raumfeste Punkt, um den die Bewegung erfolgt, bezüglich seiner Position bekannt ist. Die Bewegung des Instruments kann also um einen räumlich unbekannten, raumfesten Punkt erfolgen, und dieser raumfeste Punkt kann eine raumfeste Positionierungsaufnahme für den funktionalen Abschnitt des Instruments, insbesondere die Spitze des Instruments sein. Diese Positionierungsaufnahme wird es zwar gestatten, die Spitze festzuhalten, jedoch eine Bewegung des Instruments noch immer möglich machen.

Bei diesen Ausführungsformen der Erfindung, bei denen die Position des funktionalen Instrumentenabschnittes bzw. die Spitzenposition ermittelt wird, ergeben sich weitere Vorteile bzw. Einsatzmöglichkeiten. Eine davon ist die Weiterverwendung von Instrumenten, die leicht beschädigt oder verformt worden sind, ansonsten ihre Funktion aber noch erfüllen können. Wenn chirurgische Instrumente ihre Geometrie verändern (bei starker Beanspruchung, durch Reinigungs- oder Sterilisierungsverfahren, durch Verbiegungen, die aufgrund des Herunterfallens von Instrumenten oder ihrer Zweckentfremdung entstehen können), können sie durch den Einsatz des erfindungsgemäßen Identifizierungsverfahrens noch immer weiter verwendet werden. Es ist nämlich lediglich notwendig, die Position des funktionalen Abschnittes bzw. der Spitze des Instruments so, wie es oben beschrieben wurde, zu ermitteln, nachdem die ausgemessene Markeranordnung identifiziert und dem Instrument zugeordnet worden ist. Wie schon erwähnt ist es lediglich wichtig, dass der Marker-Starrkörper während der Verwendung starr bleibt und sich nicht gegenüber dem Instrument bewegt. Weil die Markergeometrien nicht im Navigationssystem hinterlegt sind sondern gemäß der Erfindung erst beim Einsatz bzw. am Anfang des Einsatzes des Instruments ermittelt werden, ist es mit dem erfindungsgemäßen System ausgeschlossen, dass - wie beim Stand der Technik - vordefinierte Markeranordnungen einem Instrument zugeordnet werden, das nicht mehr so aussieht, wie es gemäß den hinterlegten Daten auszusehen hat. Entsprechende Fehler werden vermieden. Aus diesem Grund können auch Instrumente, die zwar einmal hinsichtlich ihrer Form und Markergeometrie in einer Datenbank hinterlegt waren, jedoch aufgrund von Verformungen oder Fertigungsfehlern anders aussehen als sie sollten, mit Hilfe des vorliegenden, erfindungsgemäßen Verfahrens noch verwendet werden.

Der oben angesprochene raumfeste Punkt kann bei der Umsetzung des Verfahrens nach der vorliegenden Erfindung auch eine Registrierungs-Landmarke an einem Patienten oder einem behandlungsunterstützenden Gerät sein. Wenn dem so ist, kann die Identifizierung des Instruments bzw. die Abstandsmessung und/oder die Bestimmung des Positionsverhältnisses bei der Registrierung einer Landmarke durch das Instrument erfolgen, so dass bis zu drei notwendige Schritte auf einmal erledigt werden können.

Der funktionale Abschnitt des Instruments bzw. die Spitze des Instruments kann, muss aber nicht unbedingt durch pivotierende Bewegungen um einen raumfesten Punkt erfolgen. Die Lage der Spitze relativ zu der Markeranordnung bzw. Markergeometrie kann auch durch die Verwendung einer Instrumenten-Kalibrierungsmatrix erfolgen, die ein Gerät ist, welches Anlagestellen (Punkte, Linien, Flächen) für Instrumente bereitstellt, an denen letztere registriert werden können.

Die Erfindung betrifft ferner ein Programm, das, wenn es auf einem Computer läuft oder in einem Computer geladen ist, den Computer veranlasst, ein Verfahren durchzuführen, wie es in verschiedenen Ausführungsformen oben beschrieben wurde. Sie betrifft auch ein Computerprogramm-Speichermedium, das ein solches Programm aufweist.

Die Erfindung wird im Weiteren anhand einer Ausführungsform und mittels der einzigen beiliegenden Zeichnung näher erläutert. Sie kann alle hierin beschriebenen Merkmale einzeln sowie in jedweder sinnvollen Kombination umfassen, und sie ist auch als Vorrichtung durch die funktionellen Vorrichtungsteile definierbar. Die einzige beiliegende Figur zeigt ein Instrument mit einer Markeranordnung im Umfeld eines medizintechnischen Navigations- und Trackingsystems.

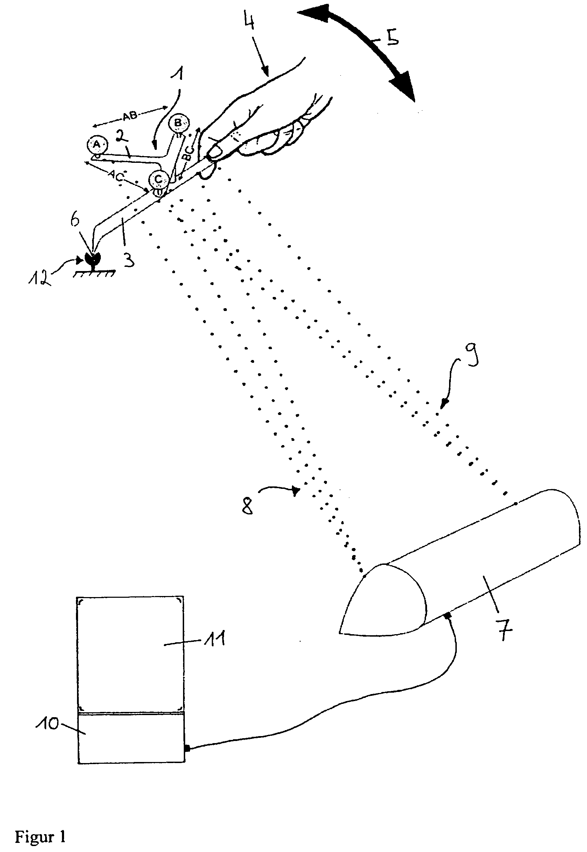

Es wird dargestellt, wie mittels eines Navigations- bzw. Trackingsystems ein starrer Körper aus Markern durch die Bewegung eines Instruments, an dem die Marker mit Hilfe einer Referenzanordnung befestigt sind, identifiziert wird. Anzumerken ist, dass die Erfindung nicht auf ein optisches Trackingsystem beschränkt ist, wie es in der Figur dargestellt wird. Dieselbe Technik kann für Ultraschall-, Magnet- oder Laser-Tracking und Navigationssysteme verwendet werden.

In der Figur ist gezeigt, wie eine Referenzanordnung 1, die an drei Armen drei Marker A, B und C trägt, an einem Instrument 3 angeordnet ist. Einer der Arme der Referenzanordnung 1 ist mit dem Bezugszeichen 2 versehen. Das Instrument 3 weist die Spitze 6 auf. Die Anordnung der Marker A, B, C an der Referenzanordnung 1 ist fest, so dass die drei Marker mit den Abständen AB, AC und BC einen starren Körper bzw. eine starre Markerkörper-Geometrie bilden. Die genannten Abstände AB, AC und BC können durch ein Trackingsystem 7 erfasst werden, das stereoskopisch arbeitet, d.h. mit zwei Kameras, deren Sichtlinien mit den Bezugszeichen 8 und 9 angedeutet sind. Wegen der stereoskopischen Abtastung durch das Trackingsystem 7 können einerseits die absoluten Positionen der Marker A, B und C bestimmt werden und andererseits natürlich auch deren Abstände. Das Trackingsystem 7 ist mit einem Navigationssystem 10 verbunden, das eine Bildschirmausgabe 11 aufweist. Die beiden letzteren Komponenten sind nur schematisch dargestellt. Das Navigationssystem 10 kann die vom Trackingsystem 7 ermittelten Positionsdaten aufnehmen, verarbeiten und über die Bildschirmausgabe 11 zur Bildunterstützung darstellen. Es führt auch die notwendigen Zuordnung und Identifizierungsmaßnahmen aus.

Mit der dargestellten Ausführungsform wird ein Weg aufgezeigt, wie man die Position der Spitze 6 des Instruments 3 erfassen kann. Hierzu wird eine Positionierungsaufnahme 12 für die Spitze des Instruments 3 bereitgestellt, die an einem raumfesten Punkt angeordnet ist, der aber seiner Position nach nicht bekannt sein muss. Die Aufnahme 12 hat im vorliegenden Beispiel eine trichterförmige Ausnehmung, welche es gestattet, die Spitze 6 festzustellen aber noch immer eine Bewegung des Instruments erlaubt. Die Bewegung wird im vorliegenden Fall pivotierend um die festgestellte Spitze 6 durchgeführt, und sie ist durch den Pfeil 5 angedeutet. Durch die Messung der Abstände zwischen den Markern A, B und C wird diese spezielle Markerkonfiguration mit den speziellen relativen Abständen der Marker als zuordnungsfähige Markeranordnung identifiziert.

Der Starrkörper, der durch die Markergeometrie vorgegeben wird, wird bestimmt. Dies kann bei der pivotierenden Bewegung um die feste Spitze 6 durchgeführt werden oder vorab bei einer anderen Bewegung des Instruments im Sichtfeld des Trackingsystems 7. Weil sich die Marker A, B, C in Kreisbewegungen bzw. in Bewegungen auf der Oberfläche einer Kugel um die Spitze 6 bewegen, kann auch das Zentrum dieser Bewegung ermittelt werden, und dieses Zentrum ist die Position der Spitze 6. Somit kann eine Zuordnung der Position der Markeranordnung zu der Position der Spitze 6 erfolgen, und im Navigationssystem 10 ist danach auch immer die Position der Spitze 6 bekannt. Das Instrument 3 kann also nach diesem Vorgang als getracktes Instrument weiter navigiert und in der bildgestützen Chirurgie eingesetzt werden, obwohl es vorab nicht in einem Datensatz des Navigationssystems 10 hinterlegt bzw. bekannt gemacht war. Anstelle der Positionsaufnahme 12 könnte auch eine Patientenlandmarke oder ein Markierungspunkt eines behandlungsunterstützenden Instrumentes gewählt werden, so dass gleichzeitig die Registrierung dieses Punktes erfolgen könnte.

Mit der vorliegenden Erfindung lassen sich jedwede, auch kostengünstig hergestellte Instrumente mit Starrkörper-Markeranordnungen für die Navigation bzw. für das Tracking verwenden. Außerdem kann die Genauigkeit vorkalibrierter Werkzeuge verbessert werden, wenn die Toleranzen dieser Werkzeuge aufgrund von Herstellungsproblemen oder aufgrund von Abnutzungserscheinungen kritisch werden.

## Patentansprüche

1. Verfahren zum Identifizieren eines Instruments (3) für die Navigation mit einem medizintechnischen Navigationssystem (10), wobei das Instrument (3) mit einer Referenzanordnung (1) versehen ist, die mehrere Marker (A, B, C) umfasst, deren Anordnung an der Referenzanordnung (1) einen starren Körper bildet, wobei die Lage der Marker zueinander nicht vorab als vordefinierte Markergeometrie für ein bestimmtes Instrument (3) im Navigationssystem hinterlegt sein muss, mit den folgenden Schritten:
- mittels eines dem Navigationssystem zugeordneten medizintechnischen, optischen Trackingsystems (7) wird der Abstand der Marker (A, B, C) zueinander gemessen,
- die Markeranordnung mit dem gemessenen Abstand für die Marker wird als eine zuordnungsfähige Markeranordnung identifiziert;
- das Positionsverhältnis des funktionalen Abschnitts des Instruments (3) zu der Markeranordnung wird durch eine Bewegung des Instruments (3) bei feststehendem funktionalen Abschnitt durch das Tracking der Marker (A, B, C) bestimmt;
- die Markeranordnung wird dem Instrument (3) zugeordnet und das Instrument (3) wird vom Navigationssystem identifiziert.

2. Verfahren nach Anspruch 1, bei dem das Instrument bei der Messung des Abstandes der Marker (A, B, C) zueinander bewegt wird, um die Markeranordnung zu identifizieren.

3. Verfahren nach Anspruch 1 oder 2, bei dem die Spitze (6) des Instruments (3) den funktionalen Abschnitt darstellt.

4. Verfahren nach Anspruch 3, bei dem die Abstandsmessung gleichzeitig mit der Bestimmung des Positionsverhältnisses erfolgt.

5. Verfahren nach Anspruch 3, bei dem die Abstandsmessung nach der Bestimmung des Positionsverhältnisses erfolgt.

6. Verfahren nach einem der Ansprüche 3 bis 5, bei dem die Bewegung des Instruments (3) bei der Bestimmung des Positionsverhältnisses um einen raumfesten Punkt erfolgt, dessen räumliche Position unbekannt ist.

7. Verfahren nach Anspruch 6, bei dem der raumfeste Punkt eine raumfeste Positionierungsaufnahme für den funktionalen Abschnitts des Instruments (3), insbesondere die Spitze (6) des Instruments (3) ist.

8. Verfahren nach Anspruch 6, bei dem der raumfeste Punkt eine Registrierungs-Landmarke an einem Patienten oder einem behandlungsunterstützenden Gerät ist.

9. Verfahren nach Anspruch 8, bei dem die Identifizierung des Instruments (3) bzw. die Abstandsmessung und/oder die Bestimmung des Positionsverhältnisses bei der Registrierung einer Landmarke durch das Instrument (3) erfolgt.

10. Verfahren nach Anspruch 1 oder 2, bei dem die Bestimmung des Positionsverhältnisses des funktionalen Abschnitts des Instruments (3), insbesondere der Spitze (6) des Instruments (3), zu der Markeranordnung durch die Verwendung einer Instrumenten-Kalibrierungsmatrix erfolgt, in der Informationen über die Gestalt des Instruments hinterlegt sind.

11. Programm, das, wenn es auf einem Computer läuft oder in einem Computer geladen ist, den Computer veranlasst, ein Verfahren gemäß einem der Ansprüche 1 bis 10 durchzuführen.

12. Computerprogramm-Speichermedium, das ein Programm nach Anspruch 11 aufweist.

## Claims

1. A method for identifying an instrument (3) for navigation using a medical navigation system (10), wherein the instrument (3) is provided with a reference array (1) which comprises a number of markers (A, B, C), the arrangement of which on the reference array (1) forms a rigid body, wherein the location of the markers with respect to each other does not have to be stored in the navigation system beforehand as a predefined marker geometry for a particular instrument (3), said method comprising the following steps:
- the distance of the markers (A, B, C) from each other is measured by means of a medical, optical tracking system (7) which is assigned to the navigation system;
- the marker array having the measured distance for the markers is identified as an assignable marker array;
- the positional relationship between the functional portion of the instrument (3) and the marker array is determined by moving the instrument (3) while the functional portion is fixed, by tracking the markers (A, B, C);
- the marker array is assigned to the instrument (3) and the instrument (3) is identified by the navigation system.

2. The method according to Claim 1, wherein the instrument is moved when measuring the distance of the markers (A, B, C) from each other, in order to identify the marker array.

3. The method according to Claim 1 or 2, wherein the tip (6) of the instrument (3) represents the functional portion.

4. The method according to Claim 3, wherein the distance is measured at the same time as determining the positional relationship.

5. The method according to Claim 3, wherein the distance is measured after the positional relationship has been determined.

6. The method according to any one of Claims 3 to 5, wherein when determining the positional relationship, the instrument (3) is moved about a spatially fixed point, the spatial position of which is unknown.

7. The method according to Claim 6, wherein the spatially fixed point is a spatially fixed positioning recess for the functional portion of the instrument (3), in particular the tip (6) of the instrument (3).

8. The method according to Claim 6, wherein the spatially fixed point is a registration landmark on a patient or treatment-assisting apparatus.

9. The method according to Claim 8, wherein the instrument (3) is identified and/or the distance measured and/or the positional relationship determined when a landmark is registered by the instrument (3).

10. The method according to Claim 1 or 2, wherein the positional relationship between the functional portion of the instrument (3), in particular the tip (6) of the instrument (3), and the marker array is determined by using an instrument calibration matrix in which information concerning the shape of the instrument is stored.

11. A program which, when it is running on a computer or is loaded onto a computer, causes the computer to perform a method in accordance with any one of Claims 1 to 10.

12. A computer program storage medium comprising a program according to Claim 11.

## Revendications

1. Procédé d'identification d'un instrument (3) pour la navigation à l'aide d'un système de navigation médico-technique (10), où l'instrument (3) est pourvu d'une structure de référence (1) qui comprend plusieurs marqueurs (A, B, C), dont l'arrangement sur la structure de référence (1) forme un corps rigide, où la position des marqueurs les uns par rapport aux autres ne doit pas nécessairement être préalablement stockée, en tant que géométrie de marqueur prédéfinie pour un instrument (3) spécifique, dans le système de navigation, comportant les étapes suivantes:
- la distance entre les marqueurs (A, B, C) est mesurée au moyen d'un système de suivi (7) optique médico-technique associé au système de navigation,
- l'arrangement de marqueurs présentant la distance mesurée pour les marqueurs est identifié en tant qu'arrangement de marqueurs pouvant être associé ; et la relation de position entre la partie fonctionnelle de l'instrument (3) et l'arrangement de marqueurs est définie, par un suivi des marqueurs (A, B, C), à partir d'un mouvement de l'instrument (3) lors duquel la partie fonctionnelle est fixe ;
- l'arrangement de marqueurs est associé à l'instrument (3) et l'instrument (3) est identifié par le système de navigation.

2. Procédé selon la revendication 1, lors duquel l'instrument est déplacé lors de la mesure de la distance entre les marqueurs (A, B, C), afin d'identifier l'arrangement de marqueurs.

3. Procédé selon la revendication 1 ou 2, lors duquel la pointe (6) de l'instrument (3) représente la partie fonctionnelle.

4. Procédé selon la revendication 3, lors duquel la mesure de distance s'effectue en même temps que la détermination de la relation de position.

5. Procédé selon la revendication 3, lors duquel la mesure de distance s'effectue après la détermination de la relation de position.

6. Procédé selon l'une des revendications 3 à 5, lors duquel le mouvement de l'instrument (3) lors de la détermination de la relation de position s'effectue autour d'un point fixe dans l'espace, dont la position dans l'espace n'est pas connue.

7. Procédé selon la revendication 6, lors duquel le point fixe dans l'espace est un logement de positionnement fixe dans l'espace pour la partie fonctionnelle de l'instrument (3), en particulier pour la pointe (6) de l'instrument (3).

8. Procédé selon la revendication 6, lors duquel le point fixe dans l'espace est un repère d'enregistrement sur un patient ou sur un appareil d'aide au traitement.

9. Procédé selon la revendication 8, lors duquel l'identification de l'instrument (3), respectivement la mesure de la distance et/ou la détermination de la relation de position s'effectue lors de l'enregistrement d'un repère par l'instrument (3).

10. Procédé selon la revendication 1 ou 2, lors duquel la détermination de la relation de position entre la partie fonctionnelle de l'instrument (3), en particulier de la pointe (6) de l'instrument (3), et l'arrangement de marqueurs s'effectue en utilisant une matrice de calibrage de l'instrument, dans laquelle sont stockées des informations concernant la forme de l'instrument.

11. Programme qui, lorsqu'il est exécuté sur un ordinateur ou chargé dans un ordinateur, provoque l'exécution par l'ordinateur d'un procédé selon l'une des revendications 1 à 10.

12. Support de stockage de programme d'ordinateur comportant un programme selon la revendication 11.
